(19) Europäisches Patentamt
European Patent Office
Office européen des brevets

(11) Veröffentlichungsnummer : **0 457 716 A1**

# (12) EUROPÄISCHE PATENTANMELDUNG

(21) Anmeldenummer : **91810268.2**

(22) Anmeldetag : **11.04.91**

(51) Int. Cl.$^5$ : **C07D 233/56,** A61K 31/415, C07D 249/06, C07D 249/04

(30) Priorität : **20.04.90 CH 1339/90**

(43) Veröffentlichungstag der Anmeldung : **21.11.91 Patentblatt 91/47**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Lang, Marc, Dr.**
**Rue des Ormes 6**
**F-68170 Rixheim (FR)**

## (54) Naphthalinderivate.

(57) Verbindungen der Formel I,

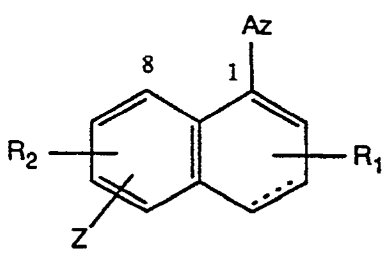

(I)

worin die punktierte Linie, Az, Z, $R_1$ und $R_2$ die in der Beschreibung angegebenen Bedeutungen haben, weisen wertvolle pharmazeutische Eigenschaften auf und sind insbesondere wirksam gegen Tumoren. Sie werden in an sich bekannter Weise hergestellt.

EP 0 457 716 A1

Jouve, 18, rue Saint-Denis, 75001 PARIS

Die Erfindung betrifft Verbindungen der Formel I,

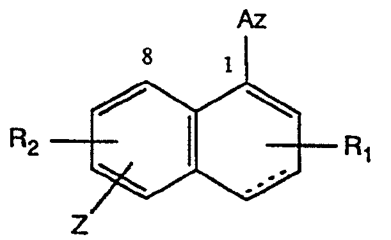

(I)

worin die punktierte Linie eine zusätzliche Bindung oder keine zusätzliche Bindung bedeutet, Az für über ein Ringstickstoffatom gebundenes Heteroaryl steht, Z einen von Wasserstoff verschiedenen Substituenten bedeutet, und $R_1$ und $R_2$ unabhängig voneinander Wasserstoff oder einen oder mehrere von Wasserstoff verschiedene Substituenten bedeuten, mit der Massgabe, dass sowohl Z als auch $R_2$ nicht einen in 8-Stellung stehenden Substituenten ausgewählt aus der Gruppe Hydroxy, unsubstituiertes oder substituiertes Alkenyloxy und unsubstituiertes oder substituiertes Alkinyloxy bedeuten, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Bedeutet die punktierte Linie in Formel I eine zusätzliche Bindung, so handelt es sich um Naphthalinderivate; bedeutet die punktierte Linie in Formel I keine zusätzliche Bindung, so ergeben sich in 1-Stellung substituierte 3,4-Dihydronaphthalinderivate.

Jeder der Reste $R_1$ und $R_2$ kann unabhängig voneinander für einen oder mehrere von Wasserstoff verschiedene Substituenten stehen. Insbesondere bedeuten die Reste $R_1$ und $R_2$ unabhängig voneinander Wasserstoff oder 1 oder 2 - vor allem 1 - von Wasserstoff verschiedene(n) Substituenten.

Die vor- und nachstehend verwendeten Allgemeinbegriffe haben im Rahmen der vorliegenden Anmeldung vorzugsweise die folgenden Bedeutungen:

Ueber ein Ringstickstoffatom gebundenes Heteroaryl steht für einen heterocyclischen Rest mit aromatischem Charakter, der mindestens ein Ringstickstoffatom enthält und über eines seiner Ringstickstoffatome gebunden ist. Bevorzugt bedeutet ein solcher Rest über ein Ringstickstoffatom gebundenes Imidazolyl oder Triazolyl, er kann aber z.B. auch für über ein Ringstickstoffatom gebundenes Tetrazolyl, Pyrazolyl, Pyrrolyl, Benzimidazolyl oder Benzotriazolyl stehen. Alle diese Reste sind vorzugsweise unsubstituiert, können aber auch substituiert sein, z.B. durch Niederalkyl, Arylniederalkyl, Trifluormethyl, Niederalkoxy, Halogen und/oder Hydroxy.

Ueber ein Ringstickstoffatom gebundenes Imidazolyl ist insbesondere 1-Imidazolyl, kann aber auch z.B. für an Ringkohlenstoffatomen, z.B. durch Niederalkyl oder Arylniederalkyl, substituiertes 1-Imidazolyl stehen.

Ueber ein Ringstickstoffatom gebundenes Triazolyl ist insbesondere 1-(1,2,4-Triazolyl), 1-(1,3,4-Triazolyl) oder 1-(1,2,3-Triazolyl), kann aber auch z.B. für 1-(1,2,5-Triazolyl) oder an Ringkohlenstoffatomen, z.B. durch Niederalkyl oder Arylniederalkyl, substituiertes 1-(1,2,4-Triazolyl), 1-(1,3,4-Triazolyl), 1-(1,2,3-Triazolyl) oder 1-(1,2,5-Triazolyl) stehen.

Ueber ein Ringstickstoffatom gebundenes Tetrazolyl ist insbesondere 1-Tetrazolyl oder 2-Tetrazolyl, kann aber auch z.B. für in 5-Stellung, z.B. durch Niederalkyl oder Arylniederalkyl, substituiertes 1- oder 2-Tetrazolyl stehen.

Ein von Wasserstoff verschiedener Substituent ist z.B. Niederalkyl, Trifluormethyl, Cycloalkyl, Arylniederalkyl, Hydroxy, Niederalkoxy, Arylniederalkoxy, Aryloxy; Acyloxy, z.B. Niederalkanoyloxy; Halogen, Amino, N-Alkylamino, N,N-Dialkylamino; Acylamino, z.B. Niederalkanoylamino; Nitro, Niederalkanoyl, Arylcarbonyl, Carboxy, Niederalkoxycarbonyl, Carbamoyl ($-CONH_2$), N-Niederalkylcarbamoyl, N,N-Diniederalkylcarbamoyl, N-Arylcarbamoyl, Cyano, Mercapto, Niederalkylthio, Niederalkylsulfonyl, Sulfamoyl ($-SO_2NH_2$), N-Niederalkylsulfamoyl oder N,N-Diniederalkylsulfamoyl.

Ein von Wasserstoff verschiedener Substituent Z ist vorzugsweise Carboxy, Niederalkoxycarbonyl, Carbamoyl, N-Niederalkylcarbamoyl, N,N-Diniederalkylcarbamoyl, N-Arylcarbamoyl, Cyano, Halogen, Hydroxy, Niederalkoxy, Arylniederalkoxy, Aryloxy, Niederalkyl, Trifluormethyl oder Arylniederalkyl.

Aryl ist z.B. Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch einen oder mehrere, insbesondere einen oder zwei, Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, Carbamoyl, N-Niederalkylcarbamoyl, N,N-Diniederalkylcarbamoyl,

Cyano, Niederalkanoyl, Arylcarbonyl, Niederalkylsulfonyl, Sulfamoyl, N-Niederalkylsulfamoyl und N,N-Diniederalkylsulfamoyl substituiert ist. In erster Linie bedeutet Aryl Phenyl, das unsubstituiert oder durch Niederalkyl, Niederalkoxy, Hydroxy, Halogen oder Trifluormethyl substituiert ist, und vor allen Dingen Phenyl.

Arylcarbonyl ist z.B. Benzoyl, welches unsubstituiert oder durch Niederalkyl, Niederalkoxy, Hydroxy, Halogen oder Trifluormethyl substituiert ist, und insbesondere Benzoyl.

Arylniederalkyl ist z.B. Phenylniederalkyl und insbesondere Benzyl.

Das Präfix "Nieder" bezeichnet einen Rest bis und mit 7, insbesondere bis und mit 4, und vor allen Dingen 1 oder 2 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Halogen steht insbesondere für Chlor und Brom, kann aber auch Fluor oder Iod bedeuten.

Niederalkanoyl ist z.B. Formyl, Acetyl, Propionyl oder Pivaloyl.

Cycloalkyl ist vorzugsweise $C_3$-$C_8$- und insbesondere $C_5$-$C_6$-Cycloalkyl, was bedeuten soll, dass es 3 bis 8 bzw. 5 bis 6 Ringkohlenstoffe enthält. Es kann aber auch substituiert sein, z.B. durch Niederalkyl.

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. Carboxy oder 1-Tetrazolyl, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die erfindungsgemässen Verbindungen der Formel I weisen wertvolle, insbesondere pharmakologisch verwendbare, Eigenschaften auf. Insbesondere hemmen sie selektiv das Enzym Aromatase bei Säugern einschliesslich Menschen. Dadurch wird die metabolische Umwandlung von Androgenen zu Oestrogenen gehemmt. Die Verbindungen der Formel I sind daher z.B. zur Behandlung Oestrogen-abhängiger Krankheiten, einschliesslich Oestrogen-abhängigem Brustkebs, insbesondere bei postmenopausalen Frauen, geeignet. Ferner sind sie z.B. nützlich bei der Behandlung von Gynäkomastie, d.h. männlicher Brustentwicklung, weil die Aromatisierung der Steroide gehemmt wird.

Diese Wirkungen können durch in vitro-Versuche oder in vivo-Versuche, vorzugsweise an Säugetieren, z.B. Meerschweinchen, Mäusen, Ratten, Katzen, Hunden oder Affen, nachgewiesen werden. Die angewandte Dosierung liegt z.B. in einem Bereich von etwa 0,001 und 10 mg/kg, vorzugsweise zwischen 0,001 und 1 mg/kg.

Die in vitro-Hemmung der Aromataseaktivität kann z.B. mit der Methode, die in J. Biol. Chem. 249, 5364 (1974) beschrieben ist, gezeigt werden. Ferner können $IC_{50}$-Werte für die Aromatasehemmung z.B. in vitro aus enzymkinetischen Studien, die die Hemmung der Umwandlung von 4-$^{14}$C-Androstendion zu 4-$^{14}$C-Oestron in menschlichen plazentalen Mikrosomen betreffen, erhalten werden. Die $IC_{50}$-Werte der erfindungsgemässen Verbindungen liegen minimal etwa bei $10^{-9}$ M.

In vivo kann die Aromatasehemmung z.B. durch die Unterdrückung des ovarialen Oestrogengehalts weiblicher Ratten gezeigt werden, die zunächst mit Stutenserumgonadotropin und 2 Tage später mit menschlichem Choriongonadotropin injiziert werden, am folgenden Tag p.o. mit einer Verbindung der Erfindung behandelt werden und 1 h später mit Androstendion. Eine weitere Möglichkeit zur in vivo Bestimmung der Aromatasehemmung wird im folgenden beschrieben: Androstendion (30 mg/kg subkutan) wird allein bzw. zusammen mit einer Verbindung der Erfindung (oral oder subkutan) 4 Tage lang an nicht geschlechtsreife weibliche Ratten verabreicht. Nach der vierten Anwendung werden die Ratten getötet, die Uteri isoliert und gewogen. Die Aromatasehemmung wird bestimmt durch das Ausmass, in dem die durch die Verabreichung von Androstendion allein verursachte Uterushypertrophie durch die gleichzeitige Verabreichung der erfindungsgemässen Verbindung unterdrückt bzw. verringert wird. Die minimale effektive Dosis der erfindungsgemässen Verbindungen bei den in vivo Tests liegt etwa zwischen 0,001 und 1 mg/kg.

Die Antitumorwirkung, insbesondere bei Oestrogen-abhängigen Tumoren, kann in vivo z.B. bei DMBA-induzierten Mammatumoren an weiblichen Sprague-Dawley-Ratten gezeigt werden [vgl. Proc. Soc. Exp. Biol.

Med. 160, 296-301 (1979)]. Die Anwendung von erfindungsgemässen Verbindungen bewirkt eine Regression der Tumoren und unterdrückt weiterhin das Auftreten neuer Tumoren bei täglichen Dosen ab etwa 1 mg/kg p.o.

Darüberhinaus haben die Verbindungen der Formel I keine Hemmwirkung auf die Spaltung der Cholesterin-Seitenkette und induzieren keine adrenale Hypertrophie, was durch endokrine Organuntersuchungen gezeigt wird.

Aufgrund ihrer pharmakologischen Eigenschaften als äusserst selektive Inhibitoren des Enzyms Aromatase sind die Verbindungen der Formel I z.B. zur Behandlung Oestrogenabhängiger Krankheiten, wie Brusttumoren (Brustkarzinoma), Endometriosis, vorzeitigen Wehen oder endometrialen Tumoren bei Frauen oder Gynäkomastie bei Männern, geeignet.

Bevorzugt sind die Verbindungen der Formel I, worin die punktierte Linie eine zusätzliche Bindung oder keine zusätzliche Bindung bedeutet, Az für über ein Ringstickstoffatom gebundenes Imidazolyl, Triazolyl, Tetrazolyl, Pyrazolyl, Pyrrolyl, Benzimidazolyl oder Benzotriazolyl steht, wobei jeder dieser Reste unsubstituiert oder an Kohlenstoffatomen durch Niederalkyl, Arylniederalkyl, Trifluormethyl, Niederalkoxy, Halogen und/oder Hydroxy substituiert ist, Z Carboxy, Niederalkoxycarbonyl, Carbamoyl, N-Niederalkylcarbamoyl, N,N-Diniederalkylcarbamoyl, N-Arylcarbamoyl, Cyano, Halogen, Hydroxy, Niederalkoxy, Arylniederalkoxy, Aryloxy, Niederalkyl, Trifluormethyl oder Arylniederalkyl bedeutet, und $R_1$ und $R_2$ unabhängig voneinander für Wasserstoff, Niederalkyl, Trifluormethyl, $C_3$-$C_8$-Cycloalkyl, Arylniederalkyl, Hydroxy, Niederalkoxy, Arylniederalkoxy, Aryloxy, Niederalkanoyloxy, Halogen, Amino, N-Alkylamino, N,N-Dialkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl oder N,N-Diniederalkylsulfamoyl stehen; wobei Aryl für Phenyl oder Naphthyl steht, welche jeweils unsubstituiert oder durch einen oder zwei Substituenten aus der Gruppe bestehend aus Niederalkyl, Niederalkoxy, Hydroxy, Halogen und Trifluormethyl substituiert sind; mit der Massgabe, dass sowohl Z als auch $R_2$ nicht für Hydroxy in 8-Stellung stehen, und Salze davon.

Sehr bevorzugt sind die Verbindungen der Formel I, worin die punktierte Linie eine zusätzliche Bindung oder keine zusätzliche Bindung bedeutet, Az für über ein Ringstickstoffatom gebundenes Imidazolyl, Triazolyl oder Tetrazolyl steht, wobei jeder dieser Reste unsubstituiert oder an Kohlenstoffatomen durch Niederalkyl oder Arylniederalkyl substituiert ist, Z Carboxy, Niederalkoxycarbonyl, Carbamoyl, N-Niederalkylcarbamoyl, N,N-Diniederalkylcarbamoyl, N-Arylcarbamoyl, Cyano, Halogen, Hydroxy, Niederalkoxy, Arylniederalkoxy, Aryloxy, Niederalkyl, Trifluormethyl oder Arylniederalkyl bedeutet, und $R_1$ und $R_2$ unabhängig voneinander für Wasserstoff, Niederalkyl, Niederalkoxy, Hydroxy, Halogen oder Trifluormethyl stehen; wobei Aryl für Phenyl oder Naphthyl steht, welche jeweils unsubstituiert oder durch einen oder zwei Substituenten aus der Gruppe bestehend aus Niederalkyl, Niederalkoxy, Hydroxy, Halogen und Trifluormethyl substituiert sind; mit der Massgabe, dass sowohl Z als auch $R_2$ nicht für Hydroxy in 8-Stellung stehen, und Salze davon.

Besonders bevorzugt sind die Verbindungen der Formel I, worin die punktierte Linie eine zusätzliche Bindung oder keine zusätzliche Bindung bedeutet, Az für über ein Ringstickstoffatom gebundenes Imidazolyl oder Triazolyl steht, Z Carboxy, Niederalkoxycarbonyl, Carbamoyl, N-Niederalkylcarbamoyl, N,N-Diniederalkylcarbamoyl, N-Arylcarbamoyl, Cyano, Halogen, Niederalkoxy, Arylniederalkoxy, Aryloxy oder Niederalkyl bedeutet, wobei Aryl jeweils für Phenyl steht, welches unsubstituiert oder durch einen oder zwei Substituenten aus der Gruppe bestehend aus Niederalkyl, Niederalkoxy, Hydroxy, Halogen und Trifluormethyl substituiert ist, und $R_1$ und $R_2$ für Wasserstoff stehen, und Salze davon.

Ganz besonders bevorzugt sind die Verbindungen der Formel I, worin die punktierte Linie keine zusätzliche Bindung bedeutet, Az für 1-Imidazolyl, 1-(1,2,4-Triazolyl), 1-(1,2,3-Triazolyl) oder 1-(1,3,4-Triazolyl) steht, Z in 5-, 6- oder 7-Stellung angeknüpft ist und Cyano, Carbamoyl, N-Arylcarbamoyl, Halogen, Niederalkoxy, Aryloxy oder Niederalkyl bedeutet, wobei Aryl für Phenyl steht, welches unsubstituiert oder durch Niederalkyl, Niederalkoxy oder Halogen substituiert ist, und $R_1$ und $R_2$ für Wasserstoff stehen, und Salze davon.

In erster Linie bevorzugt sind die Verbindungen der Formel I, worin die punktierte Linie keine zusätzliche Bindung bedeutet, Az für 1-Imidazolyl, 1-(1,2,4-Triazolyl) oder 1-(1,2,3-Triazolyl) steht, Z in 6-Stellung angeknüpft ist und Cyano, Carbamoyl, N-Phenylcarbamoyl, Chlor, Brom, Niederalkoxy, Phenyloxy oder Niederalkyl bedeutet, und $R_1$ und $R_2$ für Wasserstoff stehen, und Salze davon.

Vor allen Dingen bevorzugt sind die Verbindungen der Formel I, worin die punktierte Linie keine zusätzliche Bindung bedeutet, Az für 1-Imidazolyl oder 1-(1,2,4-Triazolyl) steht, Z in 6-Stellung angeknüpft ist und Cyano, Carbamoyl, Chlor oder Brom bedeutet, und $R_1$ und $R_2$ für Wasserstoff stehen, und Salze davon.

Als Untergruppen aus einer Gruppe von Verbindungen der Formel I sind jeweils hervorzuheben: (a) Verbindungen der Formel I, worin Z in 6-Stellung angeknüpft ist; (b) Verbindungen der Formel I, worin $R_1$ Wasserstoff bedeutet; (c) Verbindungen der Formel I, worin $R_1$ und $R_2$ Wasserstoff bedeuten; (d) Verbindungen der Formel I, worin die punktierte Linie keine zusätzliche Bindung bedeutet; und (e) Verbindungen der Formel

I, worin die punktierte Linie eine zusätzliche Bindung bedeutet und Az für 1- oder 2-Tetrazolyl steht.

Die Erfindung betrifft vor allem die in den Beispielen beschriebenen spezifischen Verbindungen und pharmazeutisch verwendbare Salze davon.

Die Verbindungen der Formel I können in an sich bekannter Weise hergestellt werden, indem man z.B.

(a) zur Herstellung von Verbindungen der Formel I, worin die punktierte Linie keine zusätzliche Bindung bedeutet, eine Verbindung der Formel II,

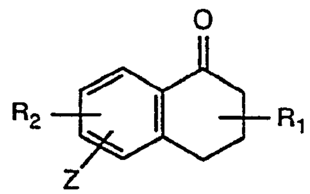

(II)

worin Z, $R_1$ und $R_2$ wie unter Formel I definiert sind, mit einer Verbindung der Formel III,

Az-H (III)

worin Az wie unter Formel I definiert ist, oder einem N-geschützten oder aktivierten Derivat davon umsetzt, oder

(b) zur Herstellung von Verbindungen der Formel I, worin die punktierte Linie keine zusätzliche Bindung bedeutet, in einer Verbindung der Formel IV,

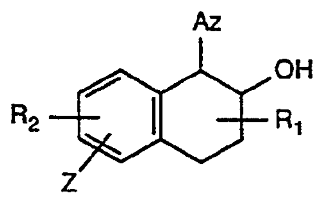

(IV)

worin Az, Z, $R_1$ und $R_2$ wie unter Formel I definiert sind, Wasser eliminiert,

(c) zur Herstellung von Verbindungen der Formel I, worin die punktierte Linie eine zusätzliche Bindung bedeutet, eine Verbindung der Formel V,

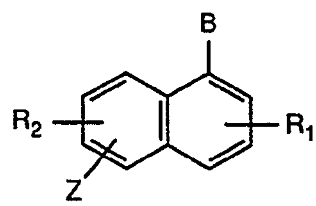

(V)

worin Z, $R_1$ und $R_2$ wie unter Formel I definiert sind und B einen über Stickstoff gebundenen Substituenten bedeutet, mit einem den Azolring Az aufbauenden Reagens umsetzt; oder

(d) zur Herstellung von Verbindungen der Formel I, worin die punktierte Linie eine zusätzliche Bindung bedeutet, eine entsprechende Verbindung der Formel I, worin die punktierte Linie keine zusätzliche Bindung bedeutet, mit einem Oxidationsmittel oxidiert; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

In der folgenden näheren Beschreibung der Verfahren (a), (b), (c) und (d) haben die Symbole Az, Z, $R_1$ und $R_2$ jeweils die unter Formel I angegebene Bedeutung, sofern nichts anderes angegeben ist.

<u>Verfahren (a):</u> Setzt man bei der Umsetzung gemäss Verfahren (a) 1,2,4-Triazol als Verbindung der Formel III ein, so erhält man - abhängig von den gewählten Reaktionsbedingungen - normalerweise Gemische von Verbindungen der Formel I, worin Az 1-(1,2,4-Triazolyl) bzw. 1-(1,3,4-Triazolyl) bedeutet, die sich z.B. chromatographisch auftrennen lassen. Setzt man entsprechend 1,2,3-Triazol als Verbindung der Formel III ein, so erhält man normalerweise Gemische von Verbindungen der Formel I, worin Az 1 -( 1,2,3-Triazolyl) bzw. 1-(1,2,5-Triazolyl) bedeutet, die sich ebenfalls z.B. chromatographisch auftrennen lassen. Setzt man entsprechend Tetrazol als Verbindung der Formel III ein, so erhält man normalerweise Gemische von Verbindungen der Formel I, worin Az 1-Tetrazolyl bzw. 2-Tetrazolyl bedeutet, die sich ebenfalls leicht z.B. durch Chromatographie auftrennen lassen.

In vielen Fällen ist es möglich, durch die Verwendung von Verbindungen der Formel III, worin ein bestimmtes Ringstickstoffatom durch eine Schutzgruppe geschützt ist, selektiv nur eine der jeweils in Frage kommenden zwei Verbindungen zu erhalten.

N-geschützte Derivate von Verbindungen der Formel III sind daher Verbindungen der Formel III, in denen ein Ringstickstoffatom durch eine geeignete Schutzgruppe, z.B. Triniederalkylsilyl, z.B. Trimethylsilyl, Niederalkanoyl, z.B. Acetyl, N,N-Diniederalkylcarbamoyl, z.B. N,N-Dimethylcarbamoyl, oder Triarylmethyl, z.B. Triphenylmethyl, geschützt ist.

Bevorzugt werden die Verbindungen der Formel III vor der Umsetzung gemäss Verfahren (a) in ein aktiviertes Derivat überführt, das eine gezielte Herstellung eines bestimmten Restes Az in den Verbindungen der Formel I ermöglicht.

Aktivierte Derivate von Verbindungen der Formel III sind z.B. die Sulfoxide der Formel Az-SO-Az, die sich z.B. nach der Umsetzung von Verbindungen der Formel III mit Thionylchlorid ergeben, insbesondere das Di-(1-imidazolyl)-sulfoxid oder das Di-[1-(1,2,4-Triazolyl)]-sulfoxid.

Die Kondensationsreaktion gemäss Verfahren (a) ist an sich bekannt und entspricht der Umsetzung eines Ketons mit einem sekundären Amin unter (formaler) Enaminbildung. Diese Reaktion wird z.B. ohne Zusatz von Basen oder vorzugsweise in Gegenwart von Basen, wie z.B. Kaliumcarbonat, Natrium, Triethylamin oder Pyridin, durchgeführt.

Die Ausgangsverbindungen der Formel II (1-Tetralone) sind an sich bekannt oder werden analog zu den bekannten Verbindungen hergestellt.

Substituierte 1-Tetralone lassen sich z.B. durch Oxidation, z.B. mit Chromtrioxid ($CrO_3$), aus den entsprechend substituierten Tetralinen der Formel VI

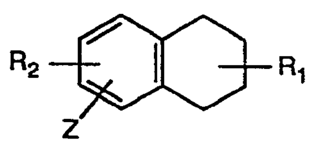

(VI)

herstellen.

Substituierte 1-Tetralone lassen sich ferner z.B. durch intramolekulare Friedel-Crafts-Acylierung von 4-Phenylbutansäuren der Formel VII

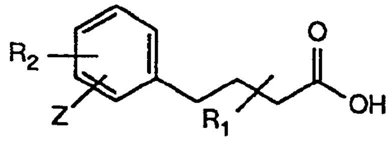

(VII)

oder Säurederivaten davon, z.B. Säurechloriden oder Säureanhydriden, herstellen. Als Katalysatoren können bei freien Säuren z.B. Polyphosphorsäure und bei Säurechloriden oder -anhydriden z.B. $AlCl_3$ verwendet werden.

Substituenten Z, $R_1$ und $R_2$, welche unter den Bedingungen der 1-Tetralonsynthesen nicht stabil sind, müssen (a) entweder vorher durch eine Schutzgruppe geschützt werden, die nach erfolgter 1-Tetralonsynthese [oder nach Durchführung des Verfahrens (a)] wieder abgespalten wird, oder (b) nach der 1-Tetralonsynthese aus anderen Gruppen, die unter den Bedingungen der 1-Tetralonsynthesen stabil sind, gemäss bekannten Umwandlungsmethoden hergestellt werden. Die beiden Methoden (a) und (b) können auch miteinander verknüpft werden: So ist es z.B. vorteilhaft, bei Aminotetralinen der Formel VI die Aminogruppen zu schützen, dann zum 1-Tetralon zu oxidieren und die Aminoschutzgruppe wieder abzuspalten. Danach kann die Aminogruppe - falls gewünscht - diazotiert und in bekannter Weise in eine Vielzahl anderer Reste Z überführt werden.

Geeignete Aminoschutzgruppen sind z.B. Acyl, etwa Niederalkanoyl, z.B. Acetyl, oder N,N-Diniederalkylcarbamoyl, z.B. N,N-Dimethylcarbamoyl; Silyl, etwa Triniederalkylsilyl, z.B. Trimethylsilyl; oder Triarylmethyl, z.B. Triphenylmethyl.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis", John Wiley & Sons, New York 1981. Charakteristisch für Schutzgruppen ist, dass sie leicht, d.h. ohne dass unerwünschte Nebenreaktionen stattfinden, z.B. solvolytisch, reduktiv oder photolytisch wieder abspaltbar sind.

Verfahren (b): Die Eliminierung von Wasser in Verbindungen der Formel IV kann entweder direkt, z.B. durch Umsetzung mit Säuren, z.B. Phosphorsäure, oder vorzugsweise indirekt über Zwischenstufen erfolgen. Beispielsweise kann die Hydroxygruppe zunächst in einen Tosyloxy-, Mesyloxy- oder Halogenrest überführt werden; nach Behandlung mit einer starken Base, z.B. Kalium-tert.-butoxid oder DBU [1,8-Diazabicyclo[5.4.0]undec-7-en (1,5-5)], erhält man die gewünschten Verbindungen der Formel I, worin die punktierte Linie keine zusätzliche Bindung bedeutet.

Die Ausgangsverbindungen der Formel IV werden z.B. durch Umsetzung einer Verbindung der Formel VIII

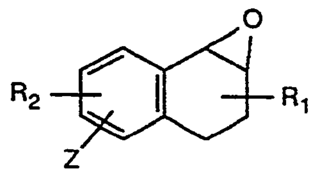

(VIII)

mit einer Verbindung der Formel III, Az-H, erhalten. Bevorzugt wird die Verbindung der Formel III dabei als Alkalimetallsalz, z.B. Natriumsalz, eingesetzt, welches z.B. durch Umsetzung von Az-H mit Natriumhydrid erhältlich ist.

Die Verbindungen der Formel VIII werden z.B. durch Epoxidierung des entsprechenden Olefins, d.h. einer Verbindung der Formel IX,

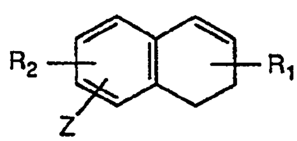

(IX)

hergestellt. Ein geeignetes Reagens dafür ist z.B. m-Chlorperbenzoesäure.

Die Verbindungen der Formel IX sind z.B. durch Wassereliminierung aus den 1-Hydroxytetralinen der Formel X

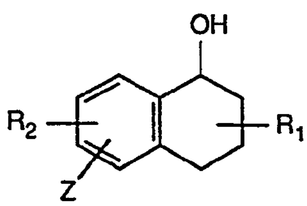

(X)

erhältlich. Die Verbindungen der Formel X ihrerseits lassen sich z.B. durch Reduktion, z.B. mit Natriumborhydrid, der entsprechenden 1-Tetralone der Formel II herstellen.

Verfahren (c): Ein über Stickstoff gebundener Substituent B ist insbesondere Amino, kann aber auch z.B. für Isocyano (-N=C), Hydrazino oder Azido stehen.

Ein den Azolring Az aufbauendes Reagens reagiert mit dem Rest B unter Bildung des gewünschten Restes Az. Dabei kann der zunächst gebildete Ring gegebenenfalls noch leicht abspaltbare Substituenten enthalten, z.B. Carboxygruppen, die sich durch einen Decarboxylierungsschritt leicht entfernen lassen. Im einzelnen lassen sich die Reste Az z.B. wie folgt aus den Substituenten B herstellen:

1. B = Isocyano; Reaktion mit Stickstoffwasserstoffsäure oder insbesondere einem Alkalimetallsalz davon, z.B. Natriumazid; ergibt Az = 1-Tetrazolyl (s. Tetrahedron Lett. 1969, 5081 )

2. B = Amino; Reaktion mit Dimethoxyethylisothiocyanat [$(CH_3O)_2CH$-$CH_2$-N=C=S] gemäss J.Chem. Soc. 127, 581 (1925), dann saure Hydrolyse z.B. mit HCl in Ethylenglykol und Entschwefelung mit Raney-Nickel (s. J. Chem. Soc. Chem. Commun. 1989, 898); ergibt Az = 1-Imidazolyl

3. B = Azido; 1,3-dipolare Cycloaddition (a) mit Acetylendicarbonsäure und Decarboxylierung der beiden Carboxygruppen oder (b) mit Acetylen [s. Bull. Soc. Chim. Belges 79, 195 (1970)]; ergibt Az = 1-(1,2,3-Triazolyl)

4. B =Hydrazino; Reaktion mit dem Iminoether $C_2H_5O-CH=NH_2^{\oplus}Cl^{\ominus}$ [s. J. Org. Chem. 37, 3504 (1972)]; ergibt Az = 1-(1,2,4-Triazolyl)

5. B = Amino; Reaktion mit (a) s-Diformylhydrazin und $ZnCl_2$ [s. Ber. 32, 797 (1899)] oder (b) N,N-Dimethylformamid-azin-hydrochlorid [$(CH_3)_2N-CH=N-N=CH-N(CH_3)_2 \cdot HCl$], gebildet durch Umsetzung von Hydrazin, DMF und Thionylchlorid [s. J. Org. Chem. 18, 1368 (1953) und J. Chem. Soc. C 1967, 1664]; ergibt 1-(1,3,4-Triazolyl).

Die Naphthalinausgangsverbindungen der Formel V sind an sich bekannt oder können analog zu den bekannnten Verbindungen hergestellt werden. Insbesondere können die 1-Aminonaphthaline der Formel V (B = $NH_2$) aus den entsprechenden 1-Tetralonen der Formel II erhalten werden, indem man letztere zunächst in die entsprechenden Oxime überführt, z.B. durch Umsetzung mit Hydroxylamin oder einem Salz davon in saurem Medium, und diese dann z.B. mit Eisessig, Essigsäureanhydrid und HCl-Gas umsetzt [s. Ber. Dtsch. Chem. Ges. 1930, 1318].

Eine weitere Möglichkeit zur Herstellung von Verbindungen der Formel V besteht in der Mononitrierung von 1- oder insbesondere 2-Naphthylamin mit konz. Schwefelsäure in Gegenwart von Harnstoff (z.B. gemäss J. Chem. Soc. 1939, 348), bei welcher 5-Nitro-1-(bzw. 2-)Naphthylamine erhalten werden. In letzteren kann die Aminogruppe diazotiert und in eine Vielzahl anderer Substituenten Z überführt werden. Schliesslich wird die Nitrogruppe durch Reduktion, z.B durch Hydrierung oder mit $SnCl_2$, in Amino umgewandelt.

Verfahren (d): Geeignete Oxidationsmittel zur Ueberführung von Dihydronaphthalinen in Naphthaline sind z.B. Mangandioxid, DDQ [2,3-Dichlor-5,6-dicyan-1,4-benzochinon], Schwefel oder Chloranil [2,3,5,6-Tetrachlor-1,4-benzochinon].

Verbindungen der Formel I können in an sich bekannter Weise in andere Verbindungen der Formel I umgewandelt werden.

Zum Beispiel können Verbindungen der Formel I, worin Z Halogen, insbesondere Brom, bedeutet, durch Umsetzung mit einem Cyanierungsmittel, z.B. Kupfer(I)cyanid, in andere Verbindungen der Formel I, worin Z Cyano bedeutet, überführt werden.

Ferner können z.B. Verbindungen der Formel I, worin Z Halogen, insbesondere Brom, bedeutet, durch Umsetzung mit Hydroxyarylverbindungen bzw. entsprechenden Alkalimetallsalzen davon, z.B. Kaliumphenolat, in andere Verbindungen der Formel I, worin Z Aryloxy bedeutet, überführt werden, vorteilhaft z.B. in Gegenwart von Kupfer.

Weiterhin können z.B. Verbindungen der Formel I, worin Z Cyano bedeutet, durch partielle Hydrolyse, z.B. mit Kaliumcarbonat und wässriger $H_2O_2$-Lösung, in andere Verbindungen der Formel I, worin Z Carbamoyl bedeutet, überführt werden.

Andererseits können z.B. auch Verbindungen der Formel I, worin Z Carbamoyl bzw. N-Niederalkylcarbamoyl bedeutet, unter Wasser- bzw. Niederalkanolabspaltung in Verbindungen der Formel I, worin Z Cyano bedeutet, überführt werden.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 200°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu

den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 0,01 % bis etwa 95 % des Wirkstoffs, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragées, Tabletten oder Kapseln, enthalten von etwa 0,5 mg bis etwa 100 mg des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 0,01 % bis 2 %, vorzugsweise ca. 0,1 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,5 mg bis etwa 100 mg, vorzugsweise von etwa 1 mg bis etwa 20 mg, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: Ether = Diethylether, Ethylacetat = Essigsäureethylester, Hexan = n-Hexan; DMSO = Dimethylsulfoxid; DC = Dünnschichtchromatographie.

Beispiel 1: 6-Cyano-1-(1-imidazolyl)-3,4-dihydronaphthalin

Eine Lösung von 272 mg Imidazol in 2,5 ml Methylenchlorid wird tropfenweise mit 175 µl Thionylchlorid versetzt. Zu der erhaltenen weissen Suspension gibt man portionsweise 342 mg 6-Cyano-1-tetralon, rührt das Reaktionsgemisch 7 h bei 40° und dann 24 h bei Raumtemperatur. Das Reaktionsgemisch wird mit 386 mg Kaliumcarbonat in 1,6 ml Wasser versetzt und mit Chloroform extrahiert. Nach dem Trocknen der organischen Phase über Natriumsulfat wird unter vermindertem Druck eingeengt. Das erhaltene Rohprodukt wird durch Säulenchromatographie (0,04-0,063 mesh $SiO_2$, Hexan/Ethylacetat 1:2) gereinigt. Man erhält nach der Kristallisation aus Ether die Titelsubstanz in Form von farblosen Kristallen; Smp. 152-153°. DC (Ethylacetat): Rf = 0,17.

Beispiel 2: 6-Cyano-1-[1-(1,2,4-triazolyl)]-3,4-dihydronaphthalin

Eine Lösung von 276 mg 1,2,4-Triazol in 2,5 ml Methylenchlorid wird tropfenweise mit 175 µl Thionylchlorid versetzt. Nach 5 min Rühren bei Raumtemperatur werden 342 mg 6-Cyano-1-tetralon zugegeben, und die orangefarbene Suspension wird weitere 6 Tage bei gleicher Temperatur gerührt. Das Reaktionsgemisch wird dann mit einer Lösung von 386 mg Kaliumcarbonat in 1,6 ml Wasser versetzt und mit Chloroform extrahiert. Die organische Phase wird abgetrennt, über Natriumsulfat getrocknet und eingeengt. Man erhält nach Säulenchromatographie ($SiO_2$, Hexan/Ethylacetat 1:2) die Titelverbindung, die aus Ether umkristallisiert wird; Smp. 169-170°. IR ($CH_2Cl_2$): 2220, 1495, 1430 $cm^{-1}$.

Beispiel 3: 6-Chlor-1-(1-imidazolyl)-3,4-dihydronaphthalin

Eine Lösung von 815 mg Imidazol in 3,2 ml Methylenchlorid wird innerhalb von 15 min mit 0,218 ml Thionylchlorid versetzt. Am Ende der exothermen Reaktion werden zur erhaltenen Suspension 451 mg 6-Chlor-1-tetralon gegeben. Nach 20 h Rühren bei Raumtemperatur werden 497 mg Kaliumcarbonat in 2 ml Wasser hinzugegeben, und es wird zweimal mit Chloroform extrahiert. Nach dem Trocknen der organischen Extrakte über Natriumsulfat wird unter vermindertem Druck eingeengt. Das erhaltene Rohprodukt wird durch Säulenchromatographie gereinigt ($SiO_2$, 0,04-0,063 mesh, 0,15 bar, Ethylacetat); Smp. (nach Kristallisation aus Ether) 68-72°.

Beispiel 4: 6-Carbamoyl-1-(1-imidazolyl)-3,4-dihydronaphthalin

6-Cyano-1-(1-imidazolyl)-3,4-dihydronaphthalin (Beispiel 1) in DMSO und $CH_2Cl_2$ wird innerhalb von 24 h mehrfach mit Kaliumcarbonat und 30 % wässriger $H_2O_2$-Lösung versetzt. Schliesslich wird Wasser hinzugegeben und der Feststoff abfiltriert. Man erhält so die Titelverbindung.

Beispiel 5: 6-Carbamoyl-1-[1-(1,2,4-triazolyl)]-3,4-dihydronaphthalin

6-Cyano-1-[1-(1,2,4-triazolyl)]-3,4-dihydronaphthalin (Beispiel 2) in DMSO und Methylenchlorid wird innerhalb von 20 h mehrfach mit Kaliumcarbonat und 30 % wässriger $H_2O_2$-Lösung versetzt. Schliesslich wird Wasser zugegeben und der Feststoff abfiltriert. Man erhält so die Titelverbindung.

Beispiel 6: 6-Brom-1-(1-imidazolyl)-3,4-dihydronaphthalin

Analog Beispiel I wird 6-Brom-1-tetralon in die Titelverbindung überführt.

Beispiel 7: 6-Brom-1-[1-(1,2,4-triazolyl)-3,4-dihydronaphthalin

Analog Beispiel 2 wird 6-Brom-1-tetralon in die Titelverbindung überführt.

Beispiel 8: 6-Cyano-1-[1-(1,2,3-triazolyl)]-3,4-dihydronaphthalin

Analog Beispiel 1 wird 6-Cyano-1-tetralon durch Umsetzung mit 1,2,3-Triazol und Thionylchlorid in die Titelverbindung überführt.

Beispiel 9: 6-Brom-1-[1-(1,2,3-triazolyl)]-3,4-dihydronaphthalin

Analog Beispiel 8 wird 6-Brom-1-tetralon in die Titelverbindung überführt.

Beispiel 10: 6-Cyano-1-(1-imidazolyl)-3,4-dihydronaphthalin

6-Brom-1-(1-imidazolyl)-3,4-dihydronaphthalin (Beispiel 6) wird durch Umsetzung mit CuCN in 1-Methylpyrrolidon in die Titelverbindung überführt, Smp. 152-153°.

Beispiel 11: 6-Brom-1-(1-imidazolyl)-3,4-dihydronaphthalin

Analog Beispiel 1 werden 2,25 g 6-Brom-1-tetralon mit 1,36 g Imidazol und 0,873 ml Thionylchlorid umgesetzt. Dabei erhält man die Titelverbindung, welche durch Säulenchromatographie ($SiO_2$, Hexan/Ethylacetat 9:1 bis 1:1) und Kristallisation aus Hexan/Ether gereinigt wird; Smp. 58-64°; $^1$H-NMR ($CDCl_3$): δ (ppm) = 0,43 (m, 2H), 2,9 (m, 2H), 6,12 (t, 2H), 6,63 (d, 1H), 7,02 (d, 1H), 7,18 (d, 1H), 7,28 (dd, 1H), 7,36 (d, 1H), 7,61 (s, 1H).

Beispiel 12:

Es werden 10000 Tabletten hergestellt, die je 5 mg Wirkstoff, z.B. eine der in den Beispielen 1-11 hergestellten Verbindungen, enthalten:

| Zusammensetzung: | |
|---|---|
| Wirkstoff | 50,00 g |
| Milchzucker | 2535,00 g |
| Maisstärke | 125,00 g |
| Polyethylenglykol 6.000 | 150,00 g |
| Magensiumstearat | 40,00 g |
| Gereinigtes Wasser | quantum satis |

Verfahren: Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Nacht bei 35°C getrocknet, durch ein Sieb mit 1,2 mm Maschenweite getrieben und zu Tabletten, welche eine Bruchrille aufweisen, gepresst.

Beispiel 13:

Es werden 1000 Kapseln hergestellt, die je 10 mg Wirkstoff, z.B. eine der in den Beispielen 1-11 hergestellten Verbindungen, enthalten:

| Zusammensetzung: | |
|---|---|
| Wirkstoff | 10,00 g |
| Milchzucker | 207,00 g |
| Modifizierte Stärke | 80,00 g |
| Magnesiumstearat | 3,00 g |

Verfahren: Alle pulverigen Bestandteile werden durch ein Sieb mit einer Maschenweite von 0,6 mm gesiebt. Dann wird der Wirkstoff in einem geeigneten Mischer zuerst mit dem Magensiumstearat und dann mit dem Milchzucker und der Stärke bis zur Homogenität vermischt. Hartgelarinekapseln Nr. 2 werden mit je 300 mg der erhaltenen Mischung unter Verwendung einer Kapselfüllmaschine gefüllt.

**Patentansprüche**

1. Verbindungen der Formel I,

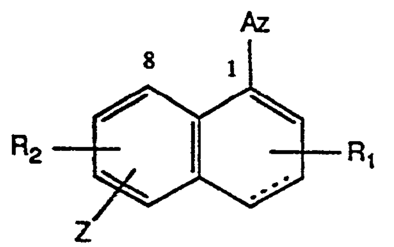

(I)

worin die punktierte Linie eine zusätzliche Bindung oder keine zusätzliche Bindung bedeutet, Az für über ein Ringstickstoffatom gebundenes Heteroaryl steht, Z einen von Wasserstoff verschiedenen Substituenten bedeutet, und $R_1$ und $R_2$ unabhängig voneinander Wasserstoff oder einen oder mehrere von Wasserstoff verschiedene Substituenten bedeuten, mit der Massgabe, dass sowohl Z als auch $R_2$ nicht einen in 8-Stellung stehenden Substituenten ausgewählt aus der Gruppe Hydroxy, unsubstituiertes oder substituiertes Alkenyloxy und unsubstituiertes oder substituiertes Alkinyloxy bedeuten, und Salze davon.

2. Verbindungen der Formel I gemäss Anspruch 1, worin die punktierte Linie eine zusätzliche Bindung oder keine zusätzliche Bindung bedeutet, Az für über ein Ringstickstoffatom gebundenes Imidazolyl, Triazolyl, Tetrazolyl, Pyrazolyl, Pyrrolyl, Benzimidazolyl oder Benzotriazolyl steht, wobei jeder dieser Reste unsubstituiert oder an Kohlenstoffatomen durch Niederalkyl, Arylniederalkyl, Trifluormethyl, Niederalkoxy, Halogen und/oder Hydroxy substituiert ist, Z Carboxy, Niederalkoxycarbonyl, Carbamoyl, N-Niederalkylcarbamoyl, N,N-Diniederalkylcarbamoyl, N-Arylcarbamoyl, Cyano, Halogen, Hydroxy, Niederalkoxy, Arylniederalkoxy, Aryloxy, Niederalkyl, Trifluormethyl oder Arylniederalkyl bedeutet, und $R_1$ und $R_2$ unabhängig voneinander für Wasserstoff, Niederalkyl, Trifluormethyl, $C_3$-$C_8$-Cycloalkyl, Arylniederalkyl, Hydroxy, Niederalkoxy, Arylniederalkoxy, Aryloxy, Niederalkanoyloxy, Halogen, Amino, N-Alkylamino, N,N-Dialkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl oder N,N-Diniederalkylsulfamoyl stehen; wobei Aryl für Phenyl oder Naphthyl steht, welche jeweils unsubstituiert oder durch einen oder zwei Substituenten aus der Gruppe bestehend aus Niederalkyl, Niederalkoxy, Hydroxy, Halogen und Trifluormethyl substituiert sind; mit der Massgabe, dass sowohl Z als auch $R_2$ nicht für Hydroxy in 8-Stellung stehen, und Salze davon.

3. Verbindungen der Formel I gemäss Anspruch 1, worin die punktierte Linie eine zusätzliche Bindung oder keine zusätzliche Bindung bedeutet, Az für über ein Ringstickstoffatom gebundenes Imidazolyl, Triazolyl oder Tetrazolyl steht, wobei jeder dieser Reste unsubstituiert oder an Kohlenstoffatomen durch Niederalkyl oder Arylniederalkyl substituiert ist, Z Carboxy, Niederalkoxycarbonyl, Carbamoyl, N-Niederalkylcarbamoyl, N,N-Diniederalkylcarbamoyl, N-Arylcarbamoyl, Cyano, Halogen, Hydroxy, Niederalkoxy, Arylniederalkoxy, Aryloxy, Niederalkyl, Trifluormethyl oder Arylniederalkyl bedeutet, und $R_1$ und $R_2$ unabhängig

voneinander für Wasserstoff, Niederalkyl, Niederalkoxy, Hydroxy, Halogen oder Trifluormethyl stehen; wobei Aryl für Phenyl oder Naphthyl steht, welche jeweils unsubstituiert oder durch einen oder zwei Substituenten aus der Gruppe bestehend aus Niederalkyl, Niederalkoxy, Hydroxy, Halogen und Trifluormethyl substituiert sind; mit der Massgabe, dass sowohl Z als auch $R_2$ nicht für Hydroxy in 8-Stellung stehen, und Salze davon.

4. Verbindungen der Formel I gemäss Anspruch 1, worin die punktierte Linie eine zusätzliche Bindung oder keine zusätzliche Bindung bedeutet, Az für über ein Ringstickstoffatom gebundenes Imidazolyl oder Triazolyl steht, Z Carboxy, Niederalkoxycarbonyl, Carbamoyl, N-Niederalkylcarbamoyl, N,N-Diniederalkylcarbamoyl, N-Arylcarbamoyl, Cyano, Halogen, Niederalkoxy, Arylniederalkoxy, Aryloxy oder Niederalkyl bedeutet, wobei Aryl jeweils für Phenyl steht, welches unsubstituiert oder durch einen oder zwei Substituenten aus der Gruppe bestehend aus Niederalkyl, Niederalkoxy, Hydroxy, Halogen und Trifluormethyl substituiert ist, und $R_1$ und $R_2$ für Wasserstoff stehen, und Salze davon.

5. Verbindungen der Formel I gemäss Anspruch 1, worin die punktierte Linie keine zusätzliche Bindung bedeutet, Az für 1-Imidazolyl, 1-(1,2,4-Triazolyl), 1-(1,2,3-Triazolyl) oder 1-(1,3,4-Triazolyl) steht, Z in 5-, 6- oder 7-Stellung angeknüpft ist und Cyano, Carbamoyl, N-Arylcarbamoyl, Halogen, Niederalkoxy, Aryloxy oder Niederalkyl bedeutet, wobei Aryl für Phenyl steht, welches unsubstituiert oder durch Niederalkyl, Niederalkoxy oder Halogen substituiert ist, und $R_1$ und $R_2$ für Wasserstoff stehen, und pharmazeutisch verwendbare Salze davon.

6. Verbindungen der Formel I gemäss Anspruch 1, worin die punktierte Linie keine zusätzliche Bindung bedeutet, Az für 1-Imidazolyl, 1-(1,2,4-Triazolyl) oder 1-(1,2,3-Triazolyl) steht, Z in 6-Stellung angeknüpft ist und Cyano, Carbamoyl, N-Phenylcarbamoyl, Chlor, Brom, Niederalkoxy, Phenyloxy oder Niederalkyl bedeutet, und $R_1$ und $R_2$ für Wasserstoff stehen, und pharmazeutisch verwendbare Salze davon.

7. Verbindungen der Formel I gemäss Anspruch 1, worin die punktierte Linie keine zusätzliche Bindung bedeutet, Az für 1-Imidazolyl oder 1-(1,2,4-Triazolyl) steht, Z in 6-Stellung angeknüpft ist und Cyano, Carbamoyl, Chlor oder Brom bedeutet, und $R_1$ und $R_2$ für Wasserstoff stehen, und pharmazeutisch verwendbare Salze davon.

8. 6-Cyano-1-(1-imidazolyl)-3,4-dihydronaphthalin gemäss Anspruch 1 oder ein pharmazeutisch verwendbares Salz davon.

9. 6-Cyano-1-[1-(1,2,4-triazolyl)]-3,4-dihydronaphthalin gemäss Anspruch 1 oder ein pharmazeutisch verwendbares Salz davon.

10. 6-Chlor-1-(1-imidazolyl)-3,4-dihydronaphthalin gemäss Anspruch 1 oder ein pharmazeutisch verwendbares Salz davon.

11. Pharmazeutische Präparate enthaltend eine Verbindung gemäss einem der Ansprüche 1 bis 10 und mindestens ein pharmazeutisch verwendbares Trägermaterial.

12. Eine Verbindung gemäss einem der Ansprüche 1 bis 10 zur Anwendung in einem Verfahren zur therapeutischen Behandlung des tierischen oder menschlichen Körpers.

13. Eine Verbindung gemäss einem der Ansprüche 1 bis 10 zur Verwendung bei der Behandlung von Krankheiten, die auf eine Hemmung des Enzyms Aromatase ansprechen.

14. Verwendung einer Verbindung gemäss einem der Ansprüche 1 bis 10 zur Herstellung pharmazeutischer Präparate.

15. Verwendung einer Verbindung gemäss einem der Ansprüche 1 bis 10 zur Herstellung pharmazeutischer Präparate für die Behandlung von Krankheiten, die auf eine Hemmung des Enzyms Aromatase ansprechen.

16. Verfahren zur Herstellung einer Verbindung der Formel I gemäss Anspruch 1, dadurch gekennzeichnet, dass man

(a) zur Herstellung von Verbindungen der Formel I, worin die punktierte Linie keine zusätzliche Bindung bedeutet, eine Verbindung der Formel II,

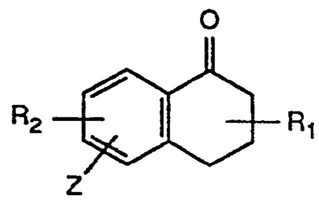

(II)

worin Z, $R_1$ und $R_2$ wie unter Formel I definiert sind, mit einer Verbindung der Formel III,

Az-H (III)

worin Az wie unter Formel I definiert ist, oder einem N-geschützten oder aktivierten Derivat davon umsetzt, oder

(b) zur Herstellung von Verbindungen der Formel I, worin die punktierte Linie keine zusätzliche Bindung bedeutet, in einer Verbindung der Formel IV,

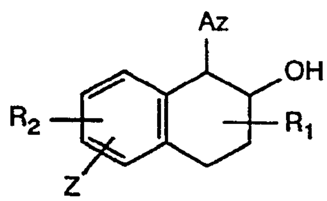

(IV)

worin Az, Z, $R_1$ und $R_2$ wie unter Formel I definiert sind, Wasser eliminiert,

(c) zur Herstellung von Verbindungen der Formel I, worin die punktierte Linie eine zusätzliche Bindung bedeutet, eine Verbindung der Formel V,

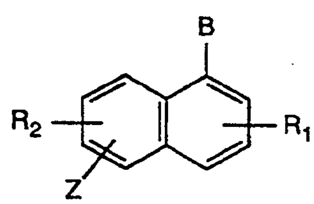

(V)

worin Z, $R_1$ und $R_2$ wie unter Formel I definiert sind und B einen über Stickstoff gebundenen Substituenten bedeutet, mit einem den Azolring Az aufbauenden Reagens umsetzt; oder

(d) zur Herstellung von Verbindungen der Formel I, worin die punktierte Linie eine zusätzliche Bindung bedeutet, eine entsprechende Verbindung der Formel I, worin die punktierte Linie keine zusätzliche Bindung bedeutet, mit einem Oxidationsmittel oxidiert; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

Europäisches Patentamt

# EUROPÄISCHER RECHERCHENBERICHT

Nummer der Anmeldung

EP 91 81 0268

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| A | EP-A-0 297 352 (BAYER AG)<br>* Seite 16; Figur Beispiel 55 * | | C 07 D 233/56<br>A 61 K 31/415<br>C 07 D 249/06<br>C 07 D 249/04 |
| A | EP-A-0 165 780 (ELI LILLY AND CO.) | | |
| A | EP-A-0 004 897 (BAYER AG) | | |
| A | EP-A-0 363 789 (FARMITALIA CARLO ERBA S.R.L.) | | |
| | | | RECHERCHIERTE SACHGEBIETE (Int. Cl.5) |
| | | | C 07 D 233/00<br>A 61 K 31/00<br>C 07 D 249/00 |

Der vorliegende Recherchenbericht wurde für alle Patentansprüche erstellt

| Recherchenort | Abschlußdatum der Recherche | Prüfer |
|---|---|---|
| DEN HAAG | 24-07-1991 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)